# EUROPEAN PATENT APPLICATION

(11) **EP 4 652 924 A1**
(43) Date of publication of application: **26.11.2025**
(21) Application number: 25178297.5
(22) Date of filing: 22.05.2025
(51) Int. Cl.: A61B 5/0245, A61B 5/11, A61B 5/256, G16H 20/30

(54) **WEARABLE DEVICE FOR THE DETECTION OF CARDIOVASCULAR FITNESS BY MEANS OF ALGORITHMS AND ASSOCIATED OPERATING METHOD**

(30) Priority: 23.05.2024 IT 202400011677
(71) Applicant: Università Cattolica del Sacro Cuore, 20123 Milano (IT); Fondazione Policlinico Universitario Agostino Gemelli IRCCS, 00168 Roma (RM) (IT)
(72) Inventor: De Spirito, Marco, 20123 Milano MI (IT); Maulucci, Giuseppe, 00168 Roma RM (IT); Serantoni, Cassandra, 00168 Roma RM (IT)
(74) Representative: Papa, Elisabetta

(57) **Abstract**

A new wearable device for monitoring the cardiovascular fitness which adapts in a customized way to the fitness profile of the user. This wearable device, provided with a microprocessor with accelerometer and gyroscope, and with a sensor for the heart rate, can be configured as smartwatch, heart rate strap, or smart shirt. The distinctive feature of the device is its capability to adapt to any physical activity, by offering a precise monitoring and feedback in real time of the cardiovascular response, by identifying four response distinct dynamic models called "Efficient adaptation", "Balance Below", "Pressure", "Active Effort" and "Proactive Preparation", by using an algorithm based on the Clustering K-Means. The system allows an accurate monitoring and feedback for training and optimizing the physical performance. This approach offers significant advantages for a wide range of users, from general health and well-being to professional sport training. Moreover, the system uses an advanced algorithm which combines Dynamic Time Warping and Clustering to evaluate the general fitness level of the user and it is capable of estimating the VO₂max value through YMCA 3 minute step test, thus providing a complete analysis of the physical capabilities of the user.

## Description

### Technical field of the invention

The present invention relates to a wearable device for the advanced detection of cardiovascular fitness of a user. Moreover, the present invention also relates to a detection process by means of the wearable device.

### Background

The evolution of the wearable devices represents a significant step towards a greater involvement of the users in their physical activities. However, the existing devices still have some limitations that hinder maximum effectiveness in optimizing individual physical performances.

In particular, most wearable devices, such as smartwatches, heart rate straps and other fitness trackers, are configured to detect and measure base parameters such as heart rate, step counting and a general estimate of burned calories. There are also some devices using more advanced algorithms to estimate fitness parameters, such as VO₂max, however, the customization of the individual physiological responses during physical activity remains a not fully achieved objective.

For example, devices having a heart rate sensor, an external workload sensor, a data processing unit and a memory are known, which allow to evaluate the physiological response of a user's body based on the intensity of the exercise and the external workload.

A customized fitness monitoring system is also known, using wearable sensors to collect data on the user motion and a processor to calculate the cardiorespiratory fitness based on the performed physical activity.

Moreover, a monitoring system for adaptive activity prescription is also known, which uses motion and biographic data to assign athletic constants to the user and thus to determine a training prescription based on the heart rate and the perceived effort.

Although these monitoring devices, or systems, offer an improvement with respect to the conventional devices, they have the drawback that they still do not succeed in providing a customized feedback, or response, in real time, in particular based on the specific physiological requirements of the user.

Moreover, even if the above-mentioned devices, or systems, represent a step forward in customized monitoring of the individual fitness, another drawback is that their effectiveness can be limited by the lack of a specific initial calibration of the user, fundamental for the subsequent customization.

### Summary of the invention

The technical problem placed and solved by the present invention then is to provide a wearable device allowing to obviate the drawbacks mentioned above with reference to the known art.

Such problem is solved by a wearable device according to claim 1.

Preferred features of the present invention are set forth in the depending claims.

The present invention provides some relevant advantages. The main advantage consists in guaranteeing an advanced detection of cardiovascular fitness with feedback, or feedback parameters, in real time and with a customization of the indications based on standardized tests and detailed analysis of the detected data, in order to optimize the individual physical performances and to improve the overall training experience.

According to an aspect of the present invention, a wearable device for detecting cardiovascular fitness of a user during a physical activity is implemented, comprising:
▪ a control unit;
▪ a plurality of sensors or transducers in communication with said control unit and including at least an accelerometer, one inertial sensor of speed and position and one or more monitoring sensors, the latter being configured to acquire at least user heart rate data.

In particular, said control unit is configured:
▪ to determine one or more performance parameters of the current activity based on the acquired data of speed, position and heart rate;
▪ to determine one or more performance adjustment parameters in real time by means of a cardiovascular response dynamic model which receives said performance parameters as input;
▪ to provide said feedback parameters to the user.

An advantage is that the feedback in real time contributes considerably to improve the training effectiveness and the motivation of the user. **In** this way even the training immersive experience is improved.

In particular, said control unit is configured to process said acquired data by the combination of a temporal analysis and hierarchical clustering algorithm, suitable for comparing and measuring similarities between series of data acquired in determined time ranges, and an unsupervised clustering algorithm, suitable for identifying specific recursive patterns computed with said performance parameters, thus defining a corresponding dynamic model.

Said performance parameters are related to specific time intervals and comprise external energy variation data and heart rate variation data, related to each other, wherein said data are projected on un scatter graph to identify said recursive patterns and to define at least four dynamic models of cardiovascular response. The external energy variation data are correlated to the data of speed and position, in particular of altitude.

More specifically, preferably the control unit is also configured:
▪ to process the acquired data by dividing the time series of heart beat and external energy into overlapped segments having a duration equal to an adaptive time window defined based on self-correlation functions of the time series of heart rate (BPM) of the user, with overlapping of a point;
▪ to extract for each segment two main performance parameters, such as:
   - variation in heart beat (ΔBPM),
   - variation in external energy (ΔE);
▪ to calculate the variation in heart beat (ΔBPM) and the variation in external energy (ΔE) as sum of a specific asymmetry index of the related segment and of a normalized value.

It is to be noted that the time ranges have a determined width and they can even be partially overlapped on each other. The width of each time range is determined by means of a self-correlation function. Moreover, the specific patterns are identified concurrently with the same time series.

This approach has the advantage that it allows to identify and isolate the different cardiovascular response models more accurately with respect to the existing methods, by allowing the user to adapt its own physical activity in real time in order to optimize the performances and to reduce the cardiovascular stress risk.

Preferably, said feedback parameters are alternatively related to:
▪ a first dynamic model, which is defined by a proportional decrease in heart rate and external energy,
▪ a second dynamic model, which is defined by a proportional increase in heart rate and external energy,
▪ a third dynamic model, which is defined by an increase in heart rate and by a decrease in external energy,
▪ a fourth dynamic model, which is defined by a decrease in heart rate and by an increase in external energy.

The first dynamic model is of "efficient adaptation", the second dynamic model is of "active effort", the third dynamic model is of "proactive preparation", and the fourth dynamic model is of "balance under pressure".

In this way, there is the advantage that through specific feedback, or feedback parameters, dedicated to each dynamic model it is possible for a user to adequate the activity intensity depending upon the respective physical and performance capabilities, and the fatigue state.

Said control unit comprises memory means to store calibration data and said acquired data, wherein said calibration data comprise parameters of individual VO₂max, identifying the maximum aerobic capacity of the user. Said control unit is configured to process said data acquired based on said calibration data.

Said control unit is operatively connected to a viewer configured to activate and signal, or display, differently each type of feedback according to the corresponding dynamic model.

According to embodiments of the present invention, said wearable device is integrated in an elastic band, or other wearable garment, and said viewer is integrated into a smartwatch which is in *wireless* communication with said control unit.

In other words, an elastic band, or another wearable garment, is provided, in which said wearable device is integrated, and wherein said viewer is integrated into a smartwatch in *wireless* communication with said control unit.

According to other embodiments of the present invention, said wearable device is integrated into a smartwatch comprising said viewer.

In other words, a smartwatch is provided, in which said wearable device is integrated. The smartwatch can comprise a pulse oximeter for collecting oxygenation data used to improve calculating the user's VO₂max.

The present invention also relates to a method of detecting advanced fitness of a user during a physical activity by means of a wearable device as described above, wherein said method provides to acquire speed, position and heart rate data respectively by means of said accelerometer, said inertial sensor, and said one or more monitoring sensors.

According to an aspect of the present invention, said method provides, by means of said control unit:
▪ to determine one or more performance parameters of the current activity based on the acquired data of speed, position and heart rate,
▪ to determine one or more performance adjustment parameters in real time by means of a corresponding dynamic model of cardiovascular response which receives said performance parameters as input;
▪ to provide said feedback parameters to the user.

In particular, the process provides, by means of the control unit, to process said acquired data by the combination of a temporal analysis and hierarchical clustering algorithm, which compares and measures similarities between series of data acquired in determined time ranges, and an unsupervised clustering algorithm, which identifies specific recursive patterns computed with said performance parameters in determined time ranges, by defining the corresponding dynamic model.

Preferably, the process provides, by means of the control unit, to analyse the dynamics in heart rate by using an adaptive time window defined based on self-correlation functions of the time series in heart rate of the user.

Specifically, preferably, the process provides to process the acquired data by means of:
▪ executing the temporal analysis algorithm through:
   - division of the time series in overlapped time ranges;
   - calculation of the self-correlation functions for the data of heart beat (BPM);
   - identification of significant periodicities in said data;
▪ applicating the hierarchical clustering algorithm by:
   - calculating the similarity measures between segments of time series;
   - creating a hierarchical structure of similar patterns;
   - determining the optimal cluster boundaries;
▪ using the unsupervised general clustering algorithm:
   - to extract features which describe the physiological response patterns;
   - to project the data on a scatter graph to display the relationships;
   - to identify at least four distinct cardiovascular response dynamic models;
▪ defining the corresponding dynamic model through:
   - integration of time and clustering results;
   - validation against performance parameters;
   - definition of thresholds and/or feedback parameters.

In particular, said feedback parameters are provided in real time on a viewer connected to said control unit, by considering alternatively:
▪ a first dynamic model, which is defined by a proportional decrease in heart rate and external energy;
▪ a second dynamic model, which is defined by a proportional increase in heart rate and external energy;
▪ a third dynamic model, which is defined by an increase in heart rate and by a decrease in external energy;
▪ a fourth dynamic model, which is defined by a decrease in heart rate and an increase in external energy;
wherein for each dynamic model a diversified visual signal is emitted.

Moreover, the process provides an initial calibration of said wearable device, wherein parameters of individual VO₂max of the user are calculated with a YMCA Step Test or other standardized tests, stored in said memory means and used as calibration data to process said acquired data and to calculate said performance parameters.

In this way, there is the advantage of having an initial calibration based on standardized tests, such as the YMCA Step test, to adapt the monitoring to the individual physiological requirements. This is essential since the cardiovascular responses can vary significantly from person to person, and an accurate customization can improve considerably the monitoring effectiveness.

Advantageously, the versatility of the wearable device and of the process in operating in different contexts of physical activity make them suitable to a wide range of users, from the fitness professionals to the individuals involved to improve their own cardiovascular health in a customized way.

Other advantages, features and use modes of the present invention will result evident from the following detailed description of some embodiments, shown by way of example and not for limitative purposes.

### Brief description of figures

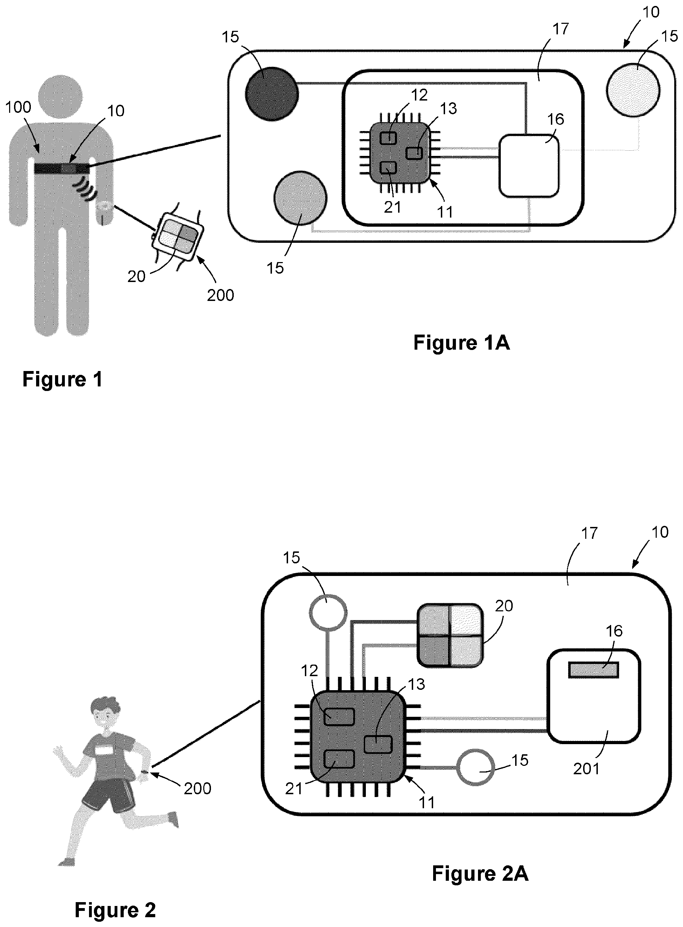

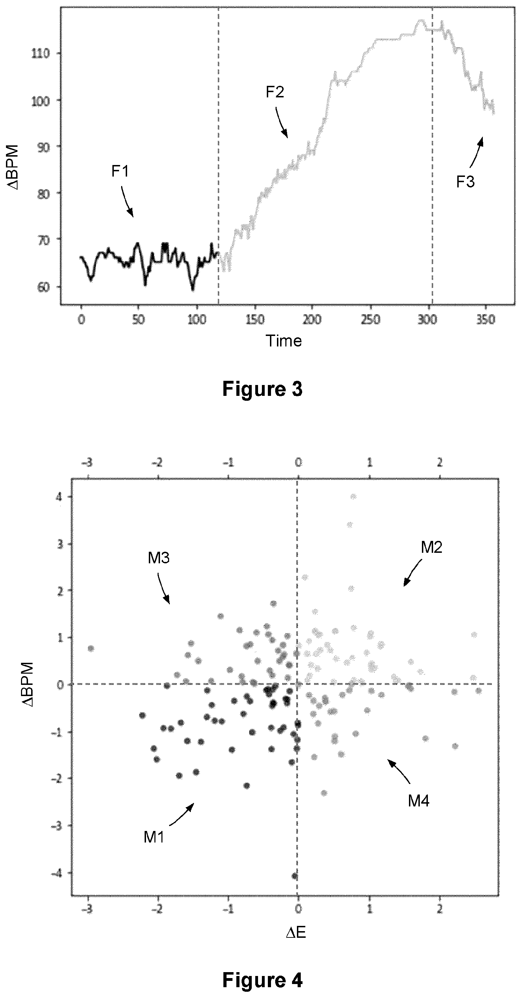

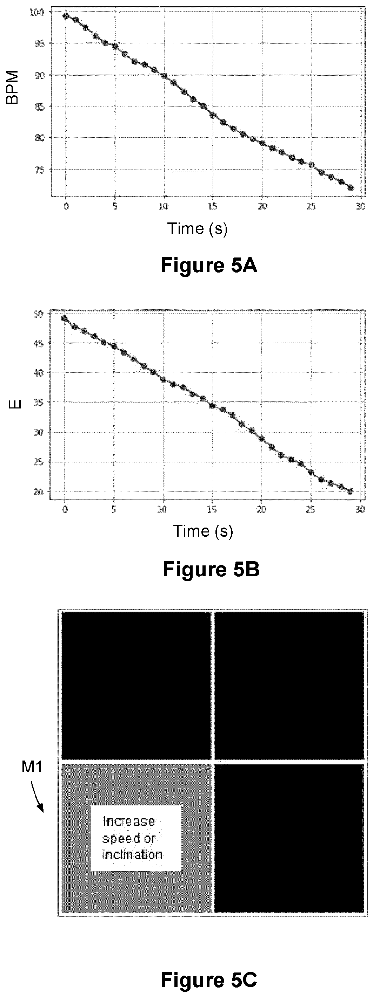

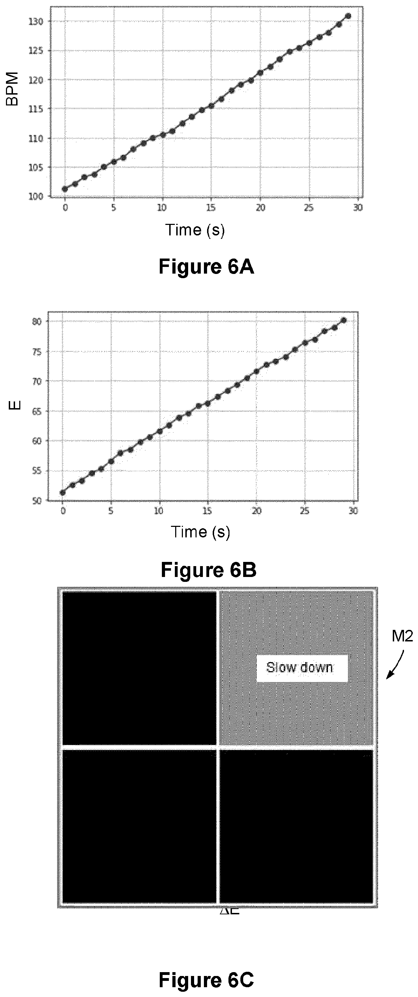

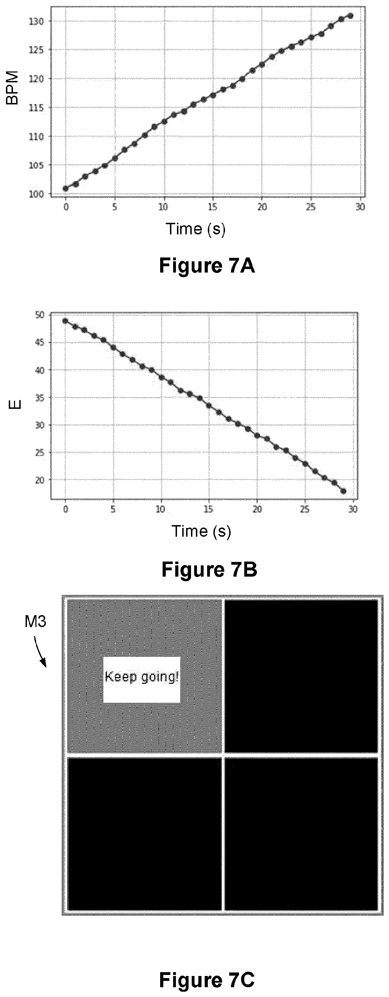

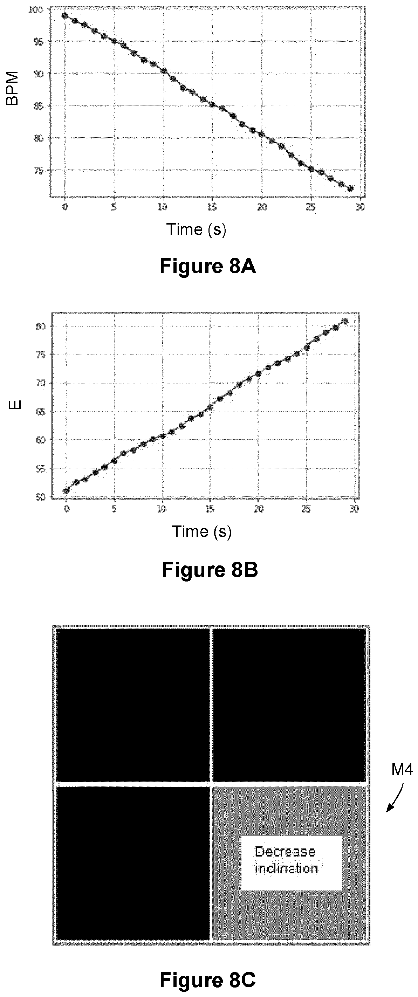

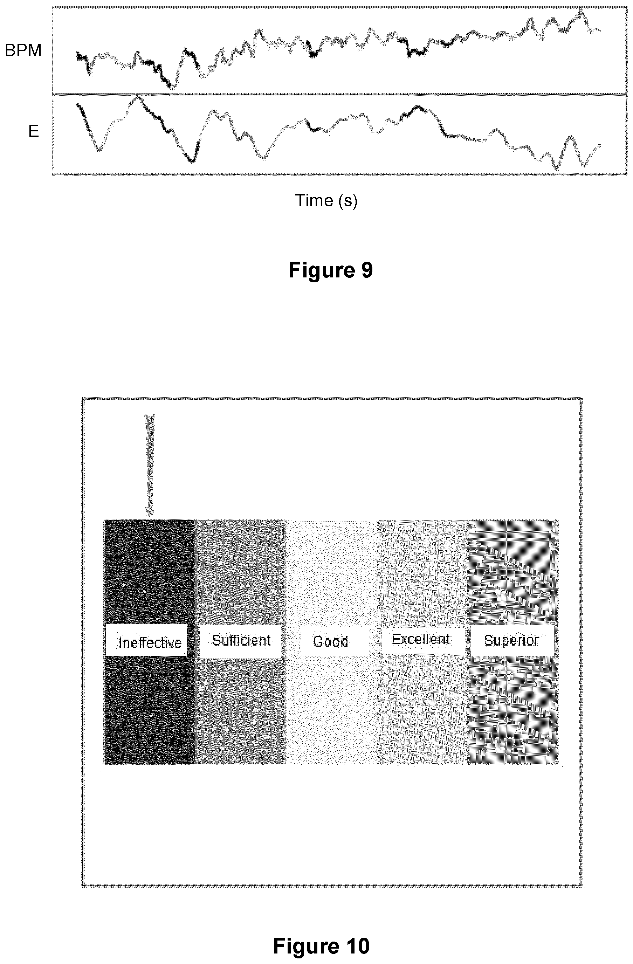

The figures of the enclosed drawings will be referred to, wherein:
▪ Figure 1 shows a schematic and simplified representation of an embodiment of a wearable device according to the present invention for the advanced detection of cardiovascular fitness by algorithms;
▪ Figure 1A shows an enlarged and out-of-scale detail of the device of Figure 1;
▪ Figure 2 shows a schematic and simplified representation of another embodiment of a wearable device according to the present invention for the advanced detection of cardiovascular fitness by algorithms;
▪ Figure 2A shows an enlarged and out-of-scale detail of the device of Figure 2;
▪ Figure 3 shows a graph of variation in heart rate in a time range during a YMCA Step Test;
▪ Figure 4 shows a scatter graph wherein external energy difference and heart rate difference data, related to determined time ranges, are put in relation;
▪ Figures 5A, 5B show two graphs identifying a first dynamic model of cardiovascular response;
▪ Figure 5C shows a graphic representation of indication based on the first dynamic model;
▪ Figures 6A, 6B show two graphs identifying a second dynamic model of cardiovascular response;
▪ Figure 6C shows a graphic representation of indication based on the second dynamic model;
▪ Figures 7A, 7B show two graphs identifying a third dynamic model of cardiovascular response;
▪ Figure 7C shows a graphic representation of indication based on the third dynamic model;
▪ Figures 8A, 8B show two graphs identifying a fourth dynamic model of cardiovascular response;
▪ Figure 8C shows a graphic representation of indication based on the fourth dynamic model;
▪ Figure 9 shows graphs of the course in heart rate and external energy spent over time during a physical activity, divided into the four dynamic models identified in the real time analysis;
▪ Figure 10 shows a graphic representation showing an evaluation of the impact of the performed exercise on the physical fitness of the user.

### Detailed description of preferred embodiments

Various embodiments and variants will be described hereinafter and this with reference to the above-illustrated figures.

Analogous components are designated in the different figures with the same numeral reference.

In the following detailed description, embodiments and variants in addition with respect to embodiments and variants already treated in the same description will be illustrated only with respect to the differences with what already illustrated.

Moreover, the different embodiments and variants described hereinafter are likely to be used in combination, where compatible.

By firstly referring to Figure 1, according to an embodiment of the invention, an advanced wearable device for detecting the advanced cardiovascular fitness of a user is designated as a whole with 10, hereinafter device 10.

The device 10 comprises at least a control unit 11 and a plurality of sensors or transducers in communication with said control unit 11. Preferably, the control unit can be a microcontroller or a microprocessor.

The sensors comprise at least an accelerometer 12 and one inertial sensor 13 to acquire speed and position data. Preferably, the inertial sensor 13 can be a gyroscope.

The device 10 even comprises one or more monitoring sensors 15 which are connected to the control unit 11 and configured to monitor and acquire data of heart rate, that is heart rate BPM, of the user.

According to embodiments, the monitoring sensors 15 can be connected to an ECG module 16 in turn connected to the control unit 11. The ECG module 16 is configured for reading the signals coming from the sensors 15 and to make usable the heart rate data to the control unit 11.

According to embodiments the device 10 can include an electronic card 17 on which the control unit 11, the accelerometer 12, the inertial sensor 13 are welded or mounted. In case, even the ECG module 16 can be welded on the electronic card 17.

The control unit 11 is configured:
▪ to determine one or more performance parameters of the current activity based on the acquired data of speed, position and heart rate;
▪ to determine one or more performance adjustment parameters in real time by means of a dynamic model of cardiovascular response which receives said performance parameters as input;
▪ to provide said feedback parameters to the user.

In particular, the control unit 11 is configured to process said acquired data by the combination of algorithms. Said algorithms comprise at least a temporal analysis and hierarchical clustering algorithm, suitable for comparing and measuring similarities between series of data acquired in determined time ranges, and an unsupervised clustering algorithm, suitable for identifying specific recursive patterns computed with said performance parameters, thus defining the corresponding dynamic model.

Preferably, the temporal analysis algorithm is a Dynamic Time Warping (DTW) algorithm. Moreover, preferably, the unsupervised clustering algorithm is a Clustering K-Means algorithm.

The performance parameters are related to specific time intervals and comprise data of variation in energy ΔE and data of variation in heart rate ΔBPM placed in relation to each other. Said data are projected on a scatter graph to identify said recursive patterns and to define at least four different dynamic models of cardiovascular response. It is to be noted that the external energy ΔE variation data are related to the data of speed and position, in particular of altitude.

Specifically, the control unit is configured even:
▪ to process the acquired data by dividing the time series of heart beat BPM and external energy E into overlapped segments having a duration equal to an adaptive time window (t_{Lag}) defined based on self-correlation functions of the time series of heart rate (BPM) of the user, with overlapping of a point;
▪ to extract for each segment two main performance parameters, such as:
   - variation in heart beat ΔBPM,
   - variation in external energy ΔE;
▪ to calculate the variation in heart beat ΔBPM and the variation in external energy ΔE as sum of a specific asymmetry index of the related segment and of a normalized value.

In particular, one defines at least:
- a first dynamic model, of "efficient adaptation", which is defined by a proportional decrease in heart rate BPM and external energy E;
- a second dynamic model, of "active effort", which is defined by a proportional increase in heart rate BPM and external energy E;
- a third dynamic model, of "proactive preparation", which is defined by an increase in heart rate BPM and by a decrease in external energy E;
- a fourth dynamic model, of "balance under pressure", which is defined by a decrease in heart rate BPM and an increase in external energy E. It is specified that the dynamic models will be defined in greater detail hereinafter.

A viewer 20 is operatively connected to the control unit 11 and it is configured to activate and signal, or display, differently each type of feedback according to the corresponding dynamic model. In other words, the feedbacks related to the first dynamic model, the feedbacks related to the second dynamic model, the feedbacks related to the third dynamic model and the feedbacks related to the fourth dynamic model are signalled differently.

According to possible embodiments, illustrated in Figures 1 and 1A, the device 10 is integrated in an elastic band 100, or other wearable garment. In other words, an elastic band 100, integrating the device 10, is provided.

In this case, the viewer 20 is integrated in a sports watch, or smartwatch, 200 in *wireless* communication with the control unit 11 to allow the optical signalling of the feedbacks.

According to other embodiments, illustrated in Figures 2 and 2A, the device 10 is integrated in a sports watch, or smartwatch, 200, which comprises the viewer 20. In other words, a smartwatch 200 which integrates the device 10, is provided. For example, the smartwatch 200 can comprise a pletoseismograph 201 for collecting heart rate data.

The control unit 11 is provided with memory means 21 to store calibration data and said acquired data. The calibration data can comprise parameters of VO₂max indicating the aerobic capacity of the user and determined by means of a standardized test performed by the same user. For example, the standardized test is a YCMA Step Test, known to the persons skilled in the art.

The control unit 11 is configured to process the data acquired based on the calibration data. Advantageously this allows to customize the feedbacks on the specific capabilities of the user.

The operation of the device 10 which corresponds also to the process for detecting cardiovascular fitness of the user during the activity, comprises the following steps.

At first, the device 10 is calibrated on the aerobic and performance capacity of the user, that is in relation to VO₂max calibration data determined by the YMCA Step Test.

For example, the calibration protocol by the YMCA Step Test provides:
- acquisition phase at rest F1, or of "baseline", wherein biometric parameters of the user are acquired, in particular data related to heart rate, at rest;
- acquisition phase during exercise F2, wherein the biometric parameters during a 3-minute-long exercise are acquired, and wherein the exercise consists in going up and down an approximately 30-cm-high step;
- recovery acquisition phase F3, wherein the biometric parameters during rest after exercise are acquired, by following the same rest conditions.

An example of the biometric parameters detected during the YMCA Step Test is illustrated in Figure 3, wherein the course of the heart rate over time expressed in seconds can be seen. As illustrated in Figure 3 it is possible to observe that there is a natural increase in the heart rate (BPM) during exercise (F2) and then a gradual reduction in the recovery phase (F3).

By means of the detected biometric parameters and other physiological parameters, like age, weight and height, the VO₂max values of the user are calculated. Then, the calibration data are stored in the memory means 21 in order to be used subsequently.

The initial calibration allows to process the acquired data and to provide customized feedbacks to the user by considering her/his performance capabilities. This is a crucial and advantageous aspect of the invention, since the physiological responses during the physical activities can vary considerably from individual to individual.

During the physical activity, the process provides to acquire the speed, position and heart rate data by means of the accelerometer 12, the inertial sensor 13 and the monitoring sensors 15, respectively.

Subsequently, by means of the control unit 11, the acquired data are processed by calculating the performance parameters related to the ongoing activity, to provide to the user in real time specific performance adjustment feedbacks according to the corresponding dynamic model.

In particular, at first the data are processed by means of a temporal analysis and hierarchical clustering algorithm, which compares and measures similarities between series of the acquired data in determined time ranges. Preferably Dynamic Time Warping, or DTW, is used, which allows to measure the similarity between two time series of data which can vary in the time domain. From the processing a similarity matrix is obtained which is then processed by means of a hierarchical clustering algorithm which organizes the similar data in a hierarchical structure.

Subsequently, an unsupervised clustering algorithm is used which identifies specific recursive patterns computed with said performance parameters in time ranges having determined width, even partially superimposed to each other. The width of each time range is determined by means of the self-correlation function. Moreover, the specific patterns are identified concurrently with the same time series. The detection of said determined time ranges is obtained by means of self-correlation functions for the time series of heart rate, that is of heart beat. Preferably, the unsupervised clustering algorithm is a Clustering K-Means algorithm.

The unsupervised clustering algorithm uses as performance parameters the data of variation in external energy ΔE and the data in variation in heart rate ΔBPM, which are related to each other and projected onto a scatter graph to identify said recursive patterns and to define at least four dynamic models of cardiovascular response (Figure 4).

Preferably, the control unit provides to analyse the dynamics in heart rate BPM by using an adaptive time window t_{Lag} defined based on self-correlation functions of the time series of the user heart rate BPM.

In addition to, or to explain, what already specified previously, the control unit 11 provides to process the acquired data by:
▪ executing the temporal analysis algorithm through:
   - division of the time series into overlapped time ranges;
   - calculation of the self-correlation functions for the data of heart beat BPM;
   - identification of significant periodicities in said data;
▪ applying the hierarchical clustering algorithm by:
   - calculating the similarity measures between segments of time series;
   - creating a hierarchical structure of similar patterns;
   - determining the optimal cluster boundaries;
▪ using the unsupervised general clustering algorithm:
   - to extract features describing the physiological response patterns;
   - to project the data on a scatter graph to display the relationships;

   - to identify at least four distinct cardiovascular response dynamic models;
▪ defining the corresponding dynamic model through:
   - integration of time and clustering results;
   - validation against performance parameters;
   - definition of thresholds and/or feedback parameters.

As illustrated in Figure 4, the scatter graph shows a plurality of relation points between data of ΔE (x-axis) and ΔBPM (y-axis), wherein each point refers to a time range. From such plurality of points four quadrants are determined, wherein each quadrant identifies a dynamic model of cardiovascular response.

From such processing, the control unit 11 provides in real time, on said viewer 20, to the user specific feedback parameters for performance adjustment according to the corresponding dynamic model of cardiovascular response.

The feedback parameters can be alternatively related to the following dynamic models.

The first dynamic model M1, or of "efficient adaptation" (Figures 5A, 5B and 5C), which is characterized by a proportional decrease in heart rate BPM and requested external energy E. This first dynamic model represents an efficient cardiovascular response and of physiological comfort to the physical activity conditions, where the heart rate decreases as energy demand decreases, by expressing the capability to maintain a balance, or a measured response to the variations in the external environment.

The graph of Figure 5A on the x-axis has time, expressed in seconds, and on the y-axis the beats per minute BPM. In such graph, a decrease in heart rate BPM over time can be observed.

The graph of Figure 5B on the x-axis has time, expressed in seconds, and on y-axis the requested external energy E. In such graph a decrease in the requested external energy E over time can be observed.

Figure 5C shows by way of example a feedback provided in real time to the user in relation to the course of her/his sports activity, wherein the provided indication represents the first dynamic model M1.

The second dynamic model M2, or of "active effort" (Figures 6A, 6B and 6C), which is characterized by a proportional increase in heart rate BPM and an increase in external energy E. This second dynamic model expresses the capability of the user cardiovascular system to respond in a proactive way to the increase in the energy needs, by underlying the aspect of the active adjustment and of the effort increase.

The graph of Figure 6A on the x-axis has time, expressed in seconds, and on the y-axis the beats per minute BPM. In such graph an increase in heart rate BPM over time can be observed.

The graph of Figure 6B on the x-axis has time, expressed in seconds, and on the y-axis the requested external energy E. In such graph an increase in the requested external energy E over time can be observed.

Figure 6C shows by way of example a feedback provided in real time to the user in relation to the course of her/his sports activity, wherein the provided indication represents the second dynamic model M2.

The third dynamic model M3, or of "proactive preparation" (Figures 7A, 7B and 7C), which is characterized by an increase in heart rate BPM and by a decrease in the requested energy E. This third dynamic model is associated to a cardiovascular response oriented to the performance. Even when the external energy request decreases (for example, while running on a flat ground), the heart rate increases, by suggesting readiness for improving the performance.

The graph of Figure 7A on the x-axis has time, expressed in seconds, and on the y-axis the beats per minute BPM. In such graph an increase in heart rate BPM over time can be observed.

The graph of Figure 7B on the x-axis has time, expressed in seconds, and on the y-axis the requested external energy E. In such graph a decrease in the requested external energy E over time can be noted.

Figure 7C shows by way of example a feedback provided in real time to the user in relation to the course of her/his sports activity, wherein the provided indication represents the third dynamic model M3.

The fourth dynamic model M4, or of "proactive preparation" (Figures 8A, 8B and 8C), which is characterized by a decrease in heart rate BPM and by an increase in the requested energy E. This fourth dynamic model highlights the uniqueness of the body's response, demonstrating a kind of effectiveness, or unexpected control, by keeping low the heart rate under potentially stressing situations which would typically require an increase.

The graph of Figure 8A on the x-axis has time, expressed in seconds, and on the y-axis the beats per minute BPM. In such graph a decrease in the heart rate BPM over time can be observed.

The graph of Figure 8B on the x-axis has time, expressed in seconds, and on the y-axis the requested external energy E. In such graph and increase in the requested external energy E over time can be noted.

Figure 8C shows by way of example a feedback provided in real time to the user in relation to the course of her/his sports activity, wherein the provided indication represents the fourth dynamic model M4.

Once the physical activity is completed, the detection process provides to supply a detailed report through graphs showing the course in heart rate BPM and requested external energy E (Figure 9). As it can be observed in Figure 9, the graphs on the x-axis have time, expressed in seconds, and on the y-axis the heart rate BPM and the requested external energy E, respectively. The graphs show the correlation between beat BPM and requested external energy E in a same time range.

For example, the course can be highlighted with differentiated colours which represent the various dynamics of heart rate and requested external energy occurred in specific moments during the exercise, with reference to the dynamic models of cardiovascular response. This has the advantage of making the user to understand that her/his own heart has responded to the physical efforts in different steps of the physical activity. Such pieces of information can be displayed on the viewer 20.

Subsequently, one provides to calculate the effectiveness of the exercise performed by the user. This calculation is based on an analysis of the density of the points present in each pattern on the dispersion graph, which are defined by dynamics of heart rate during the activity, thus offering a detailed evaluation of the impact of the exercise on the user physical fitness. Such piece of information can be displayed on the viewer 20 (Figure 10). As illustrated in Figure 10, various performance levels can be represented, each one showing an evaluation of the performed activity, such as for example "insufficient", "sufficient", "good", "excellent", or "higher", and an arrow can be used to show the evaluation of the performed physical activity.

Moreover, at the end of the physical activity the process can even provide the estimation of the user VO₂max, which provides additional information about the effectiveness of the cardiorespiratory system and about the aerobic capacity.

The present invention has been sofar described with reference to preferred embodiments. It is to be meant that other embodiments belonging to the same inventive core may exist, as defined by the protective scope of the herebelow reported claims.

## Claims

1. A wearable device (10) for detecting a cardiovascular fitness of a user during a physical activity, comprising:
▪ a control unit (11);
▪ a plurality of sensors or transducers in communication with said control unit (11) and including at least an accelerometer (12), one inertial sensor (13) of speed and position and one or more monitoring sensors (15), the latter being configured to acquire at least user heart rate data,
wherein said control unit (11) is configured:
▪ to determine one or more performance parameters of the current activity based on the acquired data of speed, position and heart rate;
▪ to process said acquired data by the combination of a temporal analysis and hierarchical clustering algorithm, suitable for comparing and measuring similarities between time series of acquired data, and an unsupervised clustering algorithm, suitable for identifying specific recursive patterns computed with said performance parameters, thus defining a corresponding dynamic model;
▪ to determine one or more performance adjustment parameters in real time by means of a dynamic model of cardiovascular response which receives said performance parameters as input;
▪ to provide said feedback parameters to the user.

2. The device (10) according to claim 1 or 2, wherein said performance parameters are related to specific time intervals and comprise external energy variation data (ΔE) and heart rate variation data (ΔBPM) related to each other, wherein said control unit (11) is also configured:
▪ to process the acquired data by dividing the time series of heart beat (BPM) and external energy (E) in overlapped segments having a duration equal to an adaptive time window (t_{Lag}) defined based on self-correlation functions of the time series of the user heart beat (BPM), with overlapping of a point;
▪ to extract for each segment two main performance parameters, such as:
- variation in heart beat (ΔBPM),
- variation in external energy (ΔE);
▪ to calculate the variation in heart beat (ΔBPM) and the variation in external energy (ΔE) as sum of a specific asymmetry index of the related segment and of a normalized value.

3. The device (10) according to claim 3, wherein said feedback parameters are alternatively related to:
▪ a first dynamic model (M1), which is defined by a proportional decrease in heart rate (BPM) and external energy (E),
▪ a second dynamic model (M2), which is defined by a proportional increase in heart rate (BPM) and external energy (E),
▪ a third dynamic model (M3), which is defined by an increase in heart rate (BPM) and by a decrease in external energy (E),
▪ a fourth dynamic model (M4), which is defined by a decrease in heart rate (BPM) and an increase in external energy (E).

4. The device (10) according to any one of the preceding claims, which comprises memory means (21) suitable for storing calibration data and said acquired data, wherein said calibration data comprise parameters of VO₂max of the user, and wherein said control unit (11) is configured to process said acquired data based on said calibration data.

5. The device (10) according to any one of the preceding claims, wherein said control unit (11) is operatively connected to a viewer (20) configured to activate and signal, or display, differently each type of feedback according to the corresponding dynamic model.

6. The device (10) according to claim 5, which is integrated into an elastic band (100) or other wearable garment, and wherein said viewer (20) is integrated into a smartwatch (200) in *wireless* communication with said control unit (11).

7. The device (10) according to claim 6, which is integrated into a smartwatch (200) comprising said viewer (20).

8. A method of detecting advanced fitness of a user during a physical activity by means of a device (10) as in any one of the preceding claims, wherein said method provides:
▪ to acquire velocity, position and heart rate data respectively by means of said accelerometer (12), said inertial sensor (13), and said one or more monitoring sensors (15),
and wherein said method provides, by means of said control unit (11):
▪ to determine one or more performance parameters of the current activity based on the acquired data of speed, position and heart rate,
▪ to process said acquired data by the combination of a temporal analysis and hierarchical clustering algorithm, which compares and measures similarities between time series of acquired data, and an unsupervised clustering algorithm, which identifies specific recursive patterns computed with said performance parameters in determined time ranges, by defining a corresponding dynamic model;
▪ to determine one or more performance adjustment parameters in real time by means of the corresponding dynamic model of cardiovascular response which receives said performance parameters as input;
▪ to provide said feedback parameters to the user.

9. The method according to claim 8, which provides, by means of said control unit (11), to analyse the dynamics of heart rate (BPM) by using an adaptive time window (t_{Lag}) defined based on self-correlation functions of the time series of heart rate (BPM) of the user.

10. The method according to claim 8 or 9, wherein said performance parameters are related to specific time intervals and comprise external energy variation data (ΔE) and heart rate variation data (ΔBPM) related to each other and projected onto a scatter graph to identify said recursive patterns and to define at least four dynamic models of cardiovascular response.

11. The method according to claim 8, 9 or 10, which provides, by means of said control unit (11), to process the acquired data by:
▪ executing the time analysis algorithm through:
- division of the time series in overlapped time ranges;
- calculation of the self-correlation functions for the data of heart beat (BPM);
- identification of significant periodicities in said data;
▪ applying the hierarchical clustering algorithm by:
- calculating the similarity measures between segments of time series;
- creating a hierarchical structure of similar patterns;
- determining optimal cluster boundaries;
▪ using the unsupervised general clustering algorithm:
- to extract features describing the physiological response patterns;
- to project data on a scatter graph to display the relationships;
- to identify at least four distinct cardiovascular response dynamic models;
▪ defining the corresponding dynamic model through:
- integration of time and clustering results;
- validation against performance parameters;
- definition of thresholds and/or feedback parameters.

12. The method according to anyone of claims 8 to 11, wherein said feedback parameters are provided in real time on a viewer (20) connected to said control unit (11), by considering alternatively:
▪ a first dynamic model (M1), which is defined by a proportional decrease in heart rate (BPM) and external energy (E);
▪ a second dynamic model (M2), which is defined by a proportional increase in heart rate (BPM) and external energy (E);
▪ a third dynamic model (M3), which is defined by an increase in heart rate (BPM) and by a decrease in external energy (E);
▪ a fourth dynamic model (M4), which is defined by a decrease in heart rate (BPM) and an increase in external energy (E);
wherein for each dynamic model a diversified visual signal is emitted.

13. The method according to anyone of claims 8 to 12, which provides an initial calibration of said device (10), wherein individual VO₂max parameters of the user are calculated with a YMCA Step Test or other standardized tests, stored in said memory means (21) and used as calibration data to process said acquired data and to calculate said performance parameters.
